# EUROPEAN PATENT APPLICATION

(11) **EP 3 333 251 A1**
(43) Date of publication of application: **13.06.2018**
(21) Application number: 16203027.4
(22) Date of filing: 08.12.2016
(51) Int. Cl.: C12M 1/12, C12M 1/00, C12M 1/34, C12M 3/00, C12M 1/06

(54) **A CONVERTIBLE BIOREACTOR, A KIT, AND A METHOD FOR CONVERTING A BIOREACTOR**

(71) Applicant: Technische Universität München, 80333 München (DE)
(72) Inventor: Riegler, Peter, 81802 München (DE); Weuster-Botz, Dirk, 80634 Munich (DE)
(74) Representative: Lucke, Andreas

(57) **Abstract**

The disclosure relates to a convertible bioreactor for biotechnological processes which is configured to be operated in different modes of operation. Further, a kit for enabling a bioreactor to be operated in different modes of operation, and a method for convening a bioreactor for different modes of operation using the kit are disclosed. (Fig. 1)

## Description

The disclosure relates to a convertible bioreactor, a kit, and a method for converting a bioreactor.

### Background

A bioreactor is used for biotechnological processes, e.g. synthesis gas fermentation. Synthesis gas fermentation (also called syngas fermentation) is a microbiological process. In this process, a synthesis gas is used as a carbon and energy source and is converted into fuel and / or chemicals by microorganisms.

The microorganisms are arranged in the bioreactor and the synthesis gas is provided to the microorganisms. Several types of bioreactors are known, including a stirred-tank reactor, a bubble column reactor, a fixed-bed reactor and a trickling filter reactor. The configuration of the bioreactor has a large influence of the efficiency of the microbiological process for producing the desired compound. However, it is difficult to compare different reactor types.

### Summary

It is an object to provide improved technologies for biotechnological processes. In particular, a comparability of different reactor types shall be provided.

A convertible bioreactor according to claim 1, a kit according to claim 3, and a method for converting a bioreactor according to claim 12 are provided. Further embodiments are subject matter of dependent claims.

In one aspect, a convertible bioreactor for biotechnological processes, e.g. synthesis gas fermentation, is disclosed. The bioreactor is configured to be operated in different modes of operation. The mode of operation may depend on components used in the bioreactor. The modes of operation may include an operation as a stirred-tank reactor, a bubble column reactor, a fixed-bed reactor and / or a trickling filter reactor. The bioreactor can be converted to all four reactor configurations. It may be necessary to have a setup and / or sterilization period when switching from one type of operation to another type. During a cultivation of microorganisms, the configuration can be changed directly (without a setup and / or sterilization) from the fixed-bed reactor to the trickling filter configuration.

In another aspect, a kit for enabling a bioreactor to be operated in different modes of operation is provided. The kit comprises a pipe for inserting in the bioreactor. A lower end of the pipe is configured to hold the pipe in the bioreactor. The pipe may be cylindrical.

In yet another aspect, a method for converting a bioreactor for different modes of operation using the kit is disclosed.

The bioreactor can be used for synthesis gas fermentation. Microorganisms are provided in the bioreactor, for example in a fluid. Suitable microorganisms are mostly known as acetogens and may include *Clostridium Ijungdahlii*, *Clostridium autoethanogenum*, *Eurobacterium limosum*, *Clostridium carboxidivorans P7, Peptostreptococcus productus,* and *Butyribacterium methylotrophicum*, *Clostridium aceticum* and many more (other suitable microorganisms are disclosed in Groher A, Weuster-Botz D (2016): Comparative reaction engineering analysis of different acetogenic bacteria for gas fermentation. J Biotechnol 228: 82-94). A synthesis gas may be introduced in the bioreactor as carbon and energy source for the microorganisms. The synthesis gas may comprise hydrogen (H₂), carbon monoxide (CO) and / or carbon dioxide (CO₂). For example, the synthesis gas may be a mixture of CO:CO₂ in a ratio of 4:1. As a result of the fermentation, alcohol or other chemical products may be produced. The bioreactor is adaptable for all kinds of biological processes, including aerobic processes, anaerobic processes, autotrophic processes and heterotrophic processes. The bioreactor may be used for cultivating the microorganisms, in particular for cultivating chemolithoautotrophic microorganisms.

The bioreactor may be sterilized by steam pressure sterilization, e.g. using desalinated water.

The bioreactor may comprise a wall surrounding an inner region. A stirring device may be arranged in the inner region of the bioreactor. The stirring device may comprise a stirring shaft. A stirring element may be fixed to the stirring shaft, for example a radial flow impeller (e.g. a Rushton turbine), an axial flow impeller, a radial propeller mixer or an axial propeller mixer. A Rushton turbine is a radial flow impeller which may be used for mixing applications. The Rushton turbine comprises a flat disc with vertical blades which are vertically mounted. The blades may be formed as flat blades, concave blades or semi-circular blades. A coupling unit may be arranged in the bioreactor which is coupled to the stirring shaft and to a drive unit for driving the stirring shaft. The coupling unit may be a magnetic coupling unit. The drive unit may be an electric motor.

The bioreactor may further comprise an outlet for sample taking, e.g. a sample taking valve. The outlet may be formed in a bottom of the bioreactor or in the wall of the bioreactor.

The bioreactor may comprise means for holding a measurement device and / or a thermal device. For example, one or more openings may be formed in the wall and / or in the bottom of the bioreactor. Several openings may be arranged on the same height of the wall. Alternatively, several openings may be formed at different heights of the wall. In one embodiment, four openings are formed in the wall and four openings are formed in the bottom. Of course, other numbers and arrangements of openings are possible. The measurement device and / or the thermal device may be fixed to the bioreactor by a screw joint.

The measurement device and / or the thermal device may be inserted through an opening such that at least a portion of the measurement device and / or the thermal device, respectively, is arranged in the inner region. Hereby, the measurement device and / or the thermal device can be brought in contact with the fluid in the inner region, for example. The measurement device may comprise a pH sensor and / or a temperature sensor. The thermal device may comprise a heating device for heating the inner region and / or a cooling device for cooling the inner region. Alternatively or in addition, a heating jacket may be arranged around the bioreactor, which may provide more accurate temperature control.

A disc may be provided which may be arranged at the lower end of the pipe. The disc may have a diameter larger than a diameter of the pipe. The disc and the pipe may be two separate components. The disc may have a hole in its center. The hole may be (slightly) smaller than the pipe diameter. In this case, the disc can easily stick to the pipe at any desired height. The disc may be cut radially once, so that the material of the disc can be pulled apart to adjust the height. The disc may hold itself on the pipe, e.g. at a height of approximately 45 mm. The diameter of the disc may be adapted to a diameter of the inner region of the bioreactor such that the disc is insertable in the inner region and fixes the pipe in the bioreactor. The diameter of the disc may be slightly smaller than the diameter of the inner region, e.g. 0.5 mm smaller, 1 mm smaller or 2 mm smaller.

The lower end of the pipe may comprise one or more magnets to fix the pipe to the bioreactor. One or more further magnets with opposite magnetic polarization than the one or more magnets may be arranged on the bottom of the bioreactor such that a magnetic connection is formed between the one or more magnets and the one or more further magnets when the pipe is inserted in the bioreactor.

One or more holes may be formed in the disc, e.g. as a liquid throughput. Several holes may be arranged in a regular layout, e.g. having same distances between each other, or in an irregular layout.

The kit may further comprise a baffle. The baffle may be inserted in the inner region of the bioreactor in order to influence a flow of the fluid in the bioreactor when the fluid is stirred. In one embodiment, the baffle may be arranged on an inner surface of the wall.

The kit may further comprise an adapter for fixing a measurement device to the bioreactor. The adapter may be configured to fix the measurement device in the means of the bioreactor for holding the measurement device. For example, the adapter may be configured to fix the measurement device in the openings in the wall and / or in the bottom of the bioreactor. The kit may comprise a first adapter which is configured to fix a pH sensor to the bioreactor. The kit may comprise a second adapter which is configured to fix a temperature sensor to the bioreactor.

The kit may further comprise a further adapter for fixing a thermal device to the bioreactor. The further adapter may be configured to fix the thermal device in the means of the bioreactor for holding the thermal device. For example, the further adapter may be configured to fix the thermal device in the openings in the wall and / or in the bottom of the bioreactor. The kit may comprise a third adapter which is configured to fix a heating device to the bioreactor. The kit may comprise a fourth adapter which is configured to fix a cooling device to the bioreactor.

The kit may further comprise a gas feed and a circular sintered metal sparger, e.g. for providing the synthesis gas to the bioreactor. A flexible tube may be provided as gas feed. The tube may be made of silicone rubber. The gas feed may be arranged at the bottom of the bioreactor. In this case, the gas introduced in the bioreactor ascends in the inner region of the bioreactor. The kit may be circular.

The kit may further comprise a fixed-bed material. The fixed-bed material may provide a footing for microorganisms. The fixed-bed material may be a ceramic material, e.g. clay ceramic or glass ceramic (sintered glass). The fixed-bed material may be a porous material, e.g. a porous ceramic. Suitable fixed-bed materials are e.g. Sera Siporax and Fluval BioMax. Usually, each microorganism requires its specific fixed-bed material. The compatibility of the microorganism with the fixed-bed material should be tested before operation.

The kit may further comprise a trickling filter. The trickling filter may be arranged in an upper region of the bioreactor. For example, the trickling filter may be fixed to the stirring shaft. The trickling filter may comprise a pool for holding a liquid. In a bottom of the trickling filter, several trickle openings (also called micro openings) may be formed through which the liquid may be dispensed. The trickling filter may further comprise gas openings for a throughput of the synthesis gas. The kit may further comprise a suction unit for the trickle filter configuration in order to guarantee a constant liquid level. The liquid may be pumped out of the bioreactor continuously to provide a fixed reaction volume in the bioreactor.

Following, embodiments of the convertible bioreactor which are adapted for different modes of operation and components of the kit suitable for the specific modes of operation are further described.

In a first mode of operation, the bioreactor may be used to operate as a stirred-tank reactor. For stirred-tank operation, the stirring element is fixed to the stirring shaft. The stirring element may be provided as a Rushton turbine. The baffle is arranged on the inner surface of the wall of the bioreactor. In operation, a fluid containing the microorganisms is inserted in the bioreactor to be stirred by the stirring element and the synthesis gas is provided. One or more measurement devices and / or one or more thermal devices may be arranged at the bioreactor, either at the wall or at the bottom. For example, a pH sensor may be arranged through an opening in the wall of the bioreactor. A temperature sensor may be inserted through an opening in the bottom of the bioreactor. A heating device and / or a cooling device may be attached to the bioreactor, e.g. through openings in the bottom. A sintered metal sparger may be arranged in a lower portion of the bioreactor. A sample may be taken via a sample taking valve which is arranged at the bottom of the bioreactor.

In a second mode of operation, the bioreactor is converted to operate as a bubble column reactor, in particular designed as a gas-lift bioreactor. In this embodiment, the stirring element and the baffle are removed from the stirred-tank reactor as described above. The pipe is inserted in the bioreactor such that the pipe surrounds the stirring shaft. The pipe may comprise the disc such that the disc is arranged below the sintered metal sparger when the pipe is inserted. By the pipe, a central region inside the pipe is separated from on outer region outside the pipe. A gas feed is arranged in the bottom of the bioreactor such that e.g. the synthesis gas is driven in the outer region. This causes a stream in the fluid to enable an optimum gas exchange. By providing the pipe, an inner circulation of the gas-fluid mixture is created in the central region. An axial propeller stirrer can optionally be fixed to the stirring shaft. One or more measurement devices and / or one or more thermal devices may be arranged at the bioreactor, either at the wall or at the bottom. The pH sensor may be fixed through an opening in the wall of the bioreactor, e.g. using the first adapter. The temperature sensor may be inserted through an opening in the bottom of the bioreactor, e.g. using the second adapter. Using the second adapter, the temperature sensor can be arranged such that the sensing element does not extend through the sintered metal sparger. The sintered metal sparger may be circular. The heating device and / or the cooling device may be attached to the bioreactor, e.g. through openings in the wall. The third adapter and the fourth adapter may be used for fixing the heating device and the cooling device, respectively. A heating jacket may be used for more accurate and less invasive temperature control.

In a third mode of operation, the bioreactor is converted to operate as a fixed-bed reactor. The pipe from the bubble column (gas-lift) reactor remains in the bioreactor. The outer region between the pipe and the wall of the bioreactor is filled with a fixed-bed material. An axial propeller stirrer can be fixed to the stirring shaft. The microorganisms arrange on the fixed-bed material and immobilize thereon and / or may form a biofilm. A gaseous substrate (e.g. synthesis gas) may be provided via the gas feed. A contact of the fixed-bed material with the pH sensor and the temperature sensor should be avoided. The pH sensor and the temperature sensor are advantageously arranged at the bottom of the bioreactor, e.g. using the first adapter and the second adapter, respectively. For the heating device and the cooling device, a contact with the fixed-bed material is not harmful. The heating device and the cooling device may be arranged through the openings in the wall, e.g. using the third adapter and the fourth adapter, respectively. A heating jacket may be used for more accurate and less invasive temperature control.

In a fourth mode of operation, the bioreactor is converted to operate as a trickling filter reactor. It is advisable that the bioreactor is operated as fixed-bed reactor before changing to the trickling filter reactor operation mode. A change of the mode of operation from the fixed-bed reactor to the trickling filter reactor is possible during operation and while bioprocesses of the microorganisms continue. After the microorganisms are immobilized on the fixed-bed material, the fluid can be removed from the bioreactor. In order to remove the fluid, a suction pipe may be used. The suction pipe may be introduced through one of the openings in the wall or in the bottom of the bioreactor. Only a small amount of the fluid may be kept at the bottom of the bioreactor at a constant height. The trickling filter is arranged in an upper region of the bioreactor and may be fixed at the stirring shaft. Hereby, the trickling filter is rotatable which provides an improved liquid distribution across the fixed bed. The trickling filter is at least partially filled with a liquid which is dispensed through trickle openings in the bottom of the trickling filter. The liquid is prepared before it is added to the trickling filter such that the liquid has the proper pH and temperature. The pH sensor and the temperature sensor may remain at the bottom of the bioreactor.

Converting the bioreactor from one reactor type to another type takes only about 15 to 20 minutes. Several reactor types can be investigated for cultivating a specific microorganism. From comparison of the several types, the best reactor type can be selected for further use, in particular for industrial applications on a large scale.

The features disclosed for the convertible bioreactor and the kit are also applicable to the method for converting the bioreactor and vice versa.

### Description of embodiments

Following, embodiments of a convertible bioreactor operating in different modes of operation are described.
- Fig. 1: is a schematic representation of the bioreactor operating as stirred-tank reactor.
- Fig. 2: is a schematic representation of the bioreactor operating as bubble column (gas-lift) reactor.
- Fig. 3: is a schematic representation of the bioreactor operating as fixed-bed reactor.
- Fig. 4: is a schematic representation of the bioreactor operating as trickling filter reactor.

In the figures, like reference numerals refer to like components.

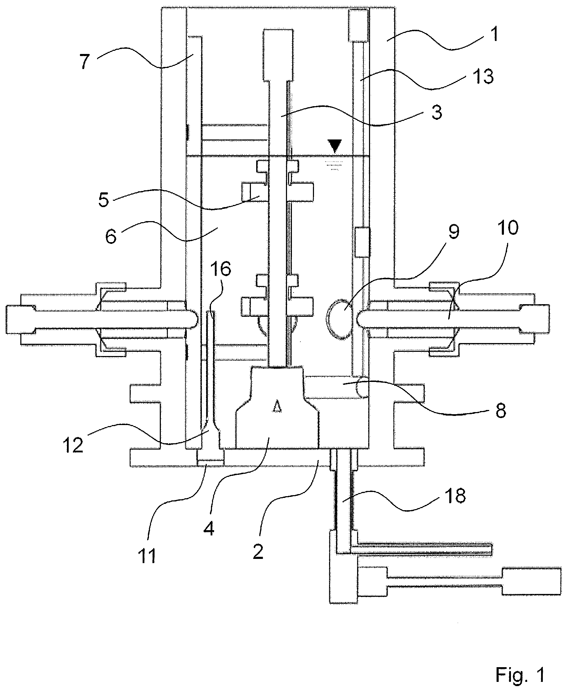

Fig. 1 shows a bioreactor operating as stirred-tank reactor. The bioreactor comprises a wall 1 and a bottom 2 which define an inner region. In the center of the bioreactor, a stirring shaft 3 is arranged which is coupled to a coupling unit 4. The coupling unit 4 may be driven by a drive unit (e.g. an electric motor, not shown) for driving the stirring shaft 3. A Rushton turbine 5 is fixed to the stirring shaft 3. A fluid 6 is filled in the bioreactor. Microorganisms may be inserted separately. A gaseous substrate (e.g. synthesis gas) is provided by a gas inlet 13. A baffle 7 is arranged at an inner surface of the wall 1. The baffle 7 influences the flow of the fluid 6 when the fluid 6 is stirred. In a lower region of the bioreactor, a sintered metal sparger 8 is arranged. In the wall 1, several openings 9 are provided for receiving a measurement device and / or a thermal device. In the embodiment of fig. 1, a pH sensor 10, is arranged in an opening 9 of the wall 1. Another opening 11 is formed in the bottom 2 of the bioreactor. A temperature sensor 12 is introduced in the bioreactor through the other opening 11 such that a sensing element 16 extends through the sintered metal sparger 8. A sample taking valve 18 is arranged at the bottom 2 of the bioreactor.

The bioreactor may have a diameter of 0.125 m and a height of 0.3 m. Four openings 9 may be formed in the wall 1, for example at a height of 0.1 m from the bottom 2. In the bottom 2 of the bioreactor, four other openings 11 may be formed, e.g. each having an M20x1.5 screw thread. The bioreactor may be closed by a reactor lid (not shown). The reactor lid may comprise a reflow cooling device, a pressure control device and a pressure control valve. The bioreactor may be operable at a pressure of 3.5 bar. The wall 1, the bottom 2 and the reactor lid may be formed of stainless steel. The sintered metal sparger 8 may have a height of 0.036 m in the bioreactor. The sintered metal sparger 8 may provide point source sparging. A stirred-tank reactor similar to the embodiment shown in Fig. 1 is commercially available, e.g. from the company Bioengineering AG. Using the kit disclosed herein, the known reactor may be converted to other reactor types enabling other modes of operation.

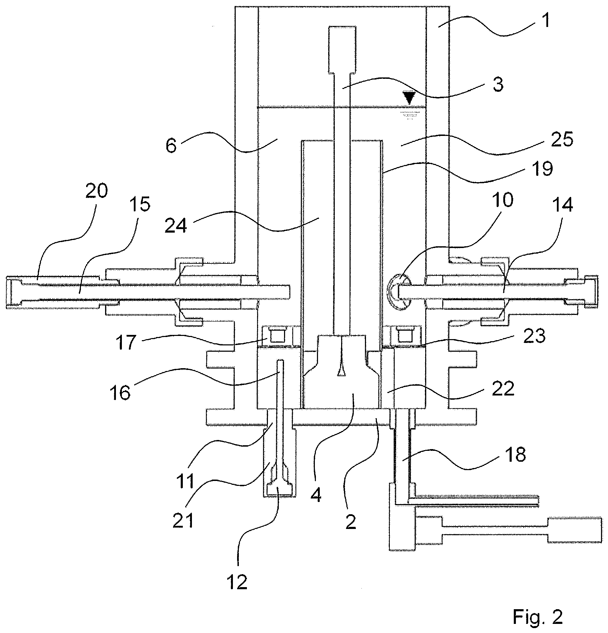

Fig. 2 shows the bioreactor converted to bubble column (gas-lift) operation. In comparison to the stirred-tank reactor shown in fig. 1, the Rushton turbine 5, the baffle 7, and the gas inlet 13 are removed. Instead, a pipe 19 is arranged around the stirring shaft 3 forming a central region 24 and an outer region 25. A disc 23 with holes is formed at a lower portion of the pipe 19. The disc 23 fixes the pipe 19 in the bioreactor. A gas feed 22 is arranged at the bottom 2 of the bioreactor. A circular sintered metal sparger 17 is arranged above the disc 23. The circular sintered metal sparger 17 may have a height of 0.045 m. A bubble column reactor with an inner circulation is formed by such arrangement of the components. The outer region 25 may also be called "riser" (fumigated volume) and the central region 24 may be called "downcomer" (non-fumigated volume). The circular sintered metal sparger 17 may provide gas over an area of 3*10⁻³ m² of the outer region (equals 32 % of the riser). The temperature sensor 12 is held by an adapter 21 at the bottom 2. By this arrangement, the length of the sensing element 16 reaching in the bioreactor is reduced. A heating device 14 and a cooling device 15 are provided in openings 9 of the wall 1. The cooling device 15 is attached to the bioreactor using another adapter 20. The bubble column (gas-lift) reactor may be operated in continuous mode.

The pipe 19 may have an outer diameter of 0.0603 m and an inner diameter of 0.0571 m. The pipe 19 may be made from stainless steel. The bubble column (gas-lift) bioreactor may have a fumigated surface (outer region) of 9.4*10⁻³ m². The inner region may be referred to as non-fumigated surface. It has an area of 2.56*10⁻³ m². This corresponds to a riser/downcomer relation of 3.68.

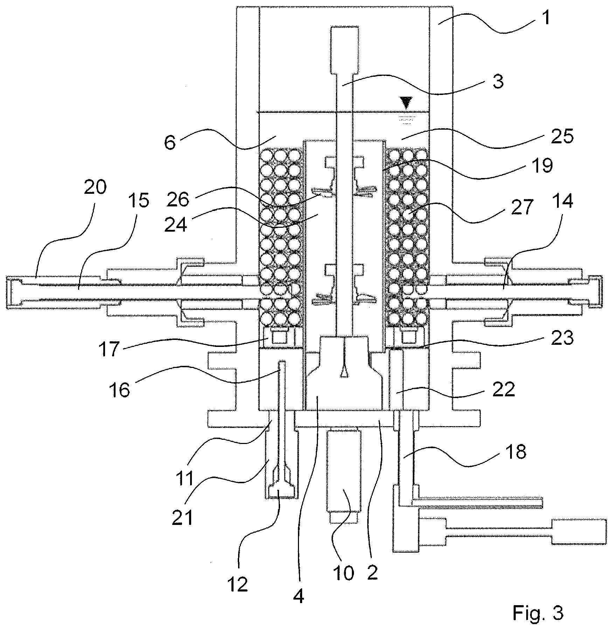

Fig. 3 shows the bioreactor of fig. 2 converted to a fixed-bed reactor (with inner circulation). An axial propeller stirrer 26 can be fixed to the stirring shaft 3 in order to provide a faster downward circulation. In addition, a fixed-bed material 27 is inserted in the outer region 25. The microorganism can immobilize on the fixed-bed material 27. The fixed-bed reactor may be operated in continuous mode.

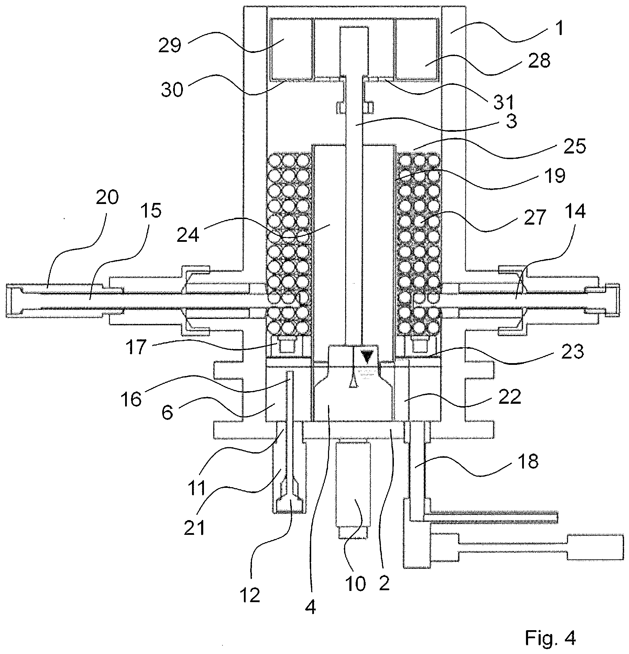

Fig. 4 shows a trickling filter reactor which is a result of a conversion of the fixed-bed reactor shown in Fig. 3. A large part of the fluid 6 is released from the bioreactor after the microorganisms are immobilized to the fixed-bed material 27. Only a small amount of fluid 6 remains at the bottom 2. A trickling filter 28 is fixed at an upper portion of the stirring shaft 3 so that the trickling filter 28 is rotatable. The trickling filter 28 comprises a pool 29 for receiving a liquid. The liquid is dispensed through trickle openings 30 formed in the bottom of the pool 29. Gas openings 31 are also formed in the trickling filter 28 for synthesis gas throughput.

The features disclosed in the specification, the claims and the figures can be relevant for the implementation of embodiments either alone or in any combination with each other.

## Claims

1. A convertible bioreactor for biotechnological processes which is configured to be operated in different modes of operation.

2. The bioreactor of claim 1, wherein the modes of operation include an operation as a stirred-tank reactor, a bubble column reactor, a fixed-bed reactor and / or a trickling filter reactor.

3. A kit for enabling a bioreactor to be operated in different modes of operation, comprising a pipe for inserting in the bioreactor, wherein a lower end of the pipe is configured to hold the pipe in the bioreactor.

4. The kit of claim 3, further comprising a disc which may be arranged at the lower end of the pipe, the disc having a diameter larger than a diameter of the pipe.

5. The kit of claim 4, wherein one or more holes are formed in the disc.

6. The kit of any one of claim 3 to 5, further comprising an adapter for fixing a measurement device to the bioreactor.

7. The kit of any one of claim 3 to 6, further comprising a further adapter for fixing a thermal device to the bioreactor.

8. The kit of any one of claim 3 to 7, further comprising a gas feed and a circular sintered metal sparger.

9. The kit of any one of claim 3 to 8, further comprising a fixed-bed material.

10. The kit of any one of claim 3 to 9, further comprising a trickling filter.

11. The kit of any one of claim 3 to 10, further comprising a baffle.

12. A method for converting a bioreactor for different modes of operation using a kit according to one of the claims 3 to 11.
